# EUROPEAN PATENT APPLICATION

(11) **EP 4 542 487 A1**
(43) Date of publication of application: **23.04.2025**
(21) Application number: 23823271.4
(22) Date of filing: 16.06.2023
(51) Int. Cl.: G06T 7/246

(54) **JAW MOVEMENT TRAJECTORY TRACKING METHOD AND APPARATUS, AND DEVICE AND MEDIUM**

(30) Priority: 17.06.2022 CN 202210692386
(71) Applicant: Shining 3D Tech Co., Ltd., Hangzhou, Zhejiang 311258 (CN)
(72) Inventor: CHEN, Xiaojun, Hangzhou, Zhejiang 311258 (CN); ZHANG, Huiquan, Hangzhou, Zhejiang 311258 (CN); XIANG, Xiaoping, Hangzhou, Zhejiang 311258 (CN); KANG, Shuaibing, Hangzhou, Zhejiang 311258 (CN)
(74) Representative: Grünecker Patent- und Rechtsanwälte PartG mbB
(86) International application number: PCT/CN2023/100827
(87) International publication number: WO 2023/241705

(57) **Abstract**

Disclosed is a method and an apparatus for tracking a motion trajectory of jaw bone, a device and a medium. The method includes: a first jaw bone three-dimensional model of a user is acquired; the motion trajectory of the preset identification point is acquired, and the identification point is disposed at jaw bone of the user; and the motion trajectory of the first jaw bone three-dimensional model is determined according to the motion trajectory of the identification point, for tracking the motion trajectory of jaw bone of the user. Based on the above method, the present disclosure realizes tracking on the jaw bone motion of the user under the premise that the identification point is disposed at jaw bone of the user, so that the use difficulty is reduced, the device is easy to mount, meanwhile, the jaw bone motion is tracked only through the identification point, so interference caused on jaw bone motion by a corresponding tracking apparatus by using a traditional jaw bone motion tracking method is avoided, and influence on the motion trajectory of the jaw bone is reduced.

## Description

The application claims priority to Chinese Patent Application No. 202210692386.1 filed on June 17, 2022 and entitled "Method and Apparatus for Tracking Motion Trajectory of Jaw Bone, Device and Medium", the disclosure of which is hereby incorporated by reference in its entirety.

### Technical Field

The present disclosure relates to the technical field of scanners, in particular to a method and an apparatus for tracking a motion trajectory of jaw bone, a device and a medium.

### Background

Three-dimensional scanners are used for various purposes, such as scanning the shape of an object, some three-dimensional scanners may be configured for detecting a motion trajectory of jaw bone, however, a tracking process of an existing three-dimensional scanner for detecting the motion trajectory of jaw bone is generally complicated, some devices may interfere with the movement of lower jaw of a user, which greatly influence the use experience, meanwhile, due to the fact that some devices are complicated and heavy, the movement of lower jaw may be interfered, and consequently, the tracking on the motion trajectory of jaw bone is influenced.

### Summary

### (1) Technical Problem to Be Solved

The embodiments of the present disclosure aim at solving the technical problem that a tracking process of an existing three-dimensional scanner for detecting the motion trajectory of jaw bone is complicated, some devices may interfere with the movement of lower jaw of a user, which greatly influence the use experience, meanwhile, due to the fact that some devices are complicated and heavy, the movement of lower jaw may be interfered, and consequently, the tracking on the motion trajectory of jaw bone is influenced.

### (2) Technical Solution

In order to solve the above technical problem, embodiments of the present disclosure provide a method and apparatus for tracking a motion trajectory of jaw bone, a device and a medium.

In a first aspect, embodiments of the present disclosure provide a method for tracking a motion trajectory of jaw bone, which includes the following operations.

A first jaw bone three-dimensional model of a user is acquired.

A motion trajectory of a preset identification point is acquired, and the identification point is disposed at jaw bone of a user.

A motion trajectory of the first jaw bone three-dimensional model of the user is determined according to the motion trajectory of the identification point, for tracking motion trajectory of jaw bone of the user.

Alternatively, before a motion trajectory of a preset identification point is acquired, the method further includes the following operations.

Position information of the identification point is acquired, and whether the position information of the identification point is matched with preset position information of the identification point is judged.

In a case that the position information of the identification point is matched with preset position information of the identification point, the step that a motion trajectory of a preset identification point is acquired is carried out.

In a case that the position information of the identification point is not matched with preset position information of the identification point, a prompt is sent out.

Alternatively, the operation that a motion trajectory of a preset identification point is acquired includes the following operations.

The identification point is scanned to obtain three-dimensional coordinates of the identification point, and the motion trajectory of the identification point is determined based on the three-dimensional coordinates of the identification point.

Alternatively, the operation that the first jaw bone three-dimensional model of the user is controlled according to the motion trajectory of the identification point includes the following operations.

The identification point and face of the user are scanned to obtain three-dimensional coordinates of the identification point and the face of the user, so as to generate a second jaw bone three-dimensional model based on the three-dimensional coordinates of the identification point and the face of the user.

The second jaw bone three-dimensional model and the first jaw bone three-dimensional model are registered.

A motion trajectory of the first jaw bone three-dimensional model is determined according to the motion trajectory of the identification point in the second jaw bone three-dimensional model.

The first jaw bone three-dimensional model is controlled according to the motion trajectory of the first jaw bone three-dimensional model.

Alternatively, after the operation that the identification point is scanned to obtain three-dimensional coordinates of the identification point, and the motion trajectory of the identification point is determined based on the three-dimensional coordinates of the identification point, the method further includes the following operation.

A display signal is sent to a preset display device so that the three-dimensional coordinates of the identification point are displayed by the display device.

Alternatively, after the operation that a motion trajectory of the first jaw bone three-dimensional model is determined according to the motion trajectory of the identification point, for tracking motion trajectory of jaw bone of the user, the method further includes the following operation.

A display signal is sent to a preset display device so that the motion trajectory of jaw bone of the user is displayed by the display device.

Alternatively, after the operation that a motion trajectory of the first jaw bone three-dimensional model is determined according to the motion trajectory of the identification point, for tracking motion trajectory of jaw bone of the user, the method further includes the following operation.

A display signal is sent to a preset display device so that two-dimensional coordinates of the identification point are displayed by the display device.

In a second aspect, the present disclosure further provides an apparatus for tracking a motion trajectory of jaw bone, which includes a first model acquiring apparatus, an identification point trajectory acquiring apparatus and a jaw bone motion tracking component.

The first model acquiring apparatus is configured to acquire a first jaw bone three-dimensional model of a user.

The identification point trajectory acquiring apparatus is configured to acquire a motion trajectory of a preset identification point, and the identification point is disposed at jaw bone of the user.

The jaw bone motion tracking component is configured to determine motion trajectory of the first jaw bone three-dimensional model according to the motion trajectory of the identification point, for tracking motion trajectory of jaw bone of the user.

In a third aspect, the present disclosure further provides an electronic device, which includes a processor and a memory.

The processor is configured to perform any one of the methods provided by the first aspect by calling a program or instructions stored in the memory.

In a fourth aspect, the present disclosure further provides a computer-readable storage medium storing a program or instruction, which enables a computer to execute any one of the methods provided by the first aspect.

### (3) Beneficial Effects

Compared with the relevant art, the technical solutions provided by the embodiments of the present disclosure have the following advantages.

According to the method for tracking a motion trajectory of jaw bone provided by the embodiments of the present disclosure, the first jaw bone three-dimensional model of the user is acquired; the motion trajectory of the preset identification point is acquired, and the identification point is disposed at jaw bone of the user; the first jaw bone three-dimensional model of the user is controlled according to the motion trajectory of the identification point, for tracking the motion trajectory of jaw bone of the user. That is, the jaw bone motion tracking method provided by the present disclosure realizes tracking for jaw bone motion of the user under the premise that the identification point is disposed at jaw bone of the user, so that use difficulty is reduced, the device is easy to mount, meanwhile, the jaw bone motion is tracked only through the identification point, so interference caused on jaw bone motion by a corresponding tracking apparatus by using a traditional jaw bone motion tracking method is avoided, and influence on the motion trajectory of the jaw bone is reduced.

It is to be understood that the above general description and the following detailed description are only exemplary and explanatory, and cannot limit the present disclosure.

### Brief Description of the Drawings

The accompanying drawings, which are incorporated in and constitute a part of this specification, illustrate the embodiments consistent with the present disclosure and, together with the specification, serve to explain the principles of the present disclosure.

In order to describe the embodiments of the present disclosure or the technical solutions in the relevant art more clearly, the drawings required to be used in descriptions about the embodiments or the relevant art will be simply introduced below, it is apparent that other drawings can further be obtained by those of ordinary skill in the art according to the drawings without creative work.
Fig. 1 is a flow diagram of a method for tracking a motion trajectory of jaw bone provided by the embodiments of the present disclosure.
Fig. 2 is a detailed flow diagram of S103 in the method shown in Fig. 1.
Fig. 3 is a schematic structure diagram of an apparatus for tracking a motion trajectory of jaw bone provided by the embodiments of the present disclosure.
Fig. 4 is a schematic diagram of a hardware structure of an electronic device provided by the embodiments of the present disclosure.

### Detailed Description of the Embodiments

In order to make the purpose, the technical solutions and the advantages of the embodiments of the present disclosure clearer, the technical solutions in the embodiments of the present disclosure will be clearly and completely described below, and it is apparent that the described embodiments are only a part rather all of embodiments of the present disclosure. All other embodiments obtained by those of ordinary skill in the art based on the embodiments of the present disclosure without creative efforts shall fall within the protection scope of the present disclosure.

Exemplary illustration will be made on a method and an apparatus for tracking a motion trajectory of jaw bone, a device and a medium provided by the embodiments of the present disclosure with reference to the accompanying drawings.

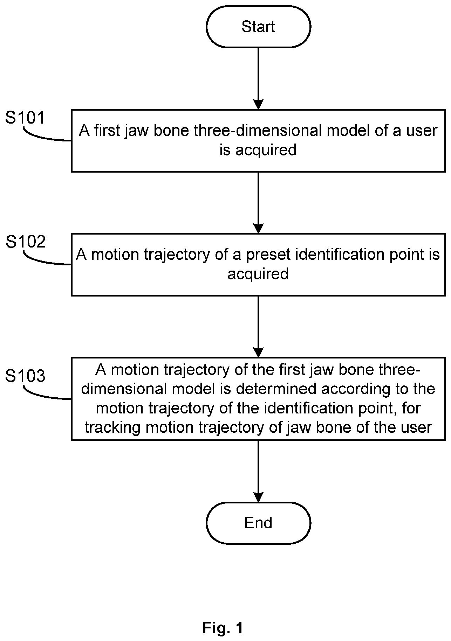

As shown in Fig. 1, Fig. 1 is a schematic flow diagram of a method for tracking a motion trajectory of jaw bone provided by the embodiments of the present disclosure, including S101-S103.

In S101, a first jaw bone three-dimensional model of a user is acquired.

In S102, a motion trajectory of a preset identification point is acquired, and the identification point is disposed at jaw bone of the user.

In S103, a motion trajectory of the first jaw bone three-dimensional model is determined according to the motion trajectory of the identification point, for tracking motion trajectory of jaw bone of the user.

In some embodiments, the first jaw bone three-dimensional model of the user may be obtained by other methods, for example, by an intraoral scanner, in the field, the first jaw bone three-dimensional model acquired by the intraoral scanner may also be referred to as a tooth three-dimensional model, the first jaw bone three-dimensional model may reflect the shape of teeth of the user and show the motion trajectory of the jaw bone of the user, etc., but due to limitation of range of a scanning window of the intraoral scanner and limitation of depth extending into mouth of the user, movement of lower jaw bone of large amplitude cannot be tracked, therefore, the embodiments of the present disclosure acquire the first jaw bone three-dimensional model only through the intraoral scanner, but does not track the motion trajectory of the jaw bone directly through the intraoral scanner, a facial scanner is not suitable for acquiring a complete first jaw bone three-dimensional model of the user, but may accurately acquire the motion trajectory of identification point of the face of the user, and therefore, the embodiments of the present disclosure acquire the motion trajectory of the identification point through the facial scanner.

In order to simplify the installation process of the device and reduce interference to the motion trajectory of jaw bone of the user, in the embodiment, a target with the identification point is fixed at the jaw bone of the user, for example, after the identification point is pasted on the target, the target is pasted at the jaw bone of the user, and the identification point faces to the front of the lower jaw, so that a camera of the facial scanner may more easily acquire the motion trajectory of the identification point As another implementation mode of the installation process, it is also possible to directly paste the identification point on the jaw bone or teeth of the user, thereby further simplifying the installation process and reducing the influence on the user.

Then the motion trajectory of the identification point is acquired through the camera, the first jaw bone three-dimensional model is controlled according to the motion trajectory of the identification point, for example, after an application program of the facial scanner is started, a user or others click a certain key on an interface of the application program, then the camera can be controlled to acquire the motion trajectory of the identification point, for example, to acquire downward motion of a certain identification point or a certain number of identification points at the lower jaw, the lower jaw of the first jaw bone three-dimensional model may be controlled to move downwards, it is to be noted that this is only an example, in an actual process, the motion trajectory of the lower jaw may be an arc, and therefore, when the lower jaw of the first jaw bone three-dimensional model is controlled to move downwards, it should also be the same as the motion trajectory of the identification point, both of which are arc-shaped.

The jaw bone motion tracking method provided by the embodiments of the present disclosure realizes tracking for jaw bone motion of the user only by disposing the identification point at jaw bone of the user, so that the use difficulty is reduced, the device is easy to mount, meanwhile, the jaw bone motion is tracked only through the identification point, so interference caused on jaw bone motion by a corresponding tracking apparatus by using a traditional jaw bone motion tracking method is avoided, and influence on the motion trajectory of the jaw bone is reduced.

In an implementation mode of the embodiments of the present disclosure, before S102, the method may further include the following operations.

Position information of the identification point is acquired, and whether the position information of the identification point is matched with preset position information of the identification point is judged.

In a case that the position information of the identification point is matched with preset position information of the identification point, the step that a motion trajectory of a preset identification point is acquired is carried out.

In a case that the position information of the identification point is not matched with preset position information of the identification point, a prompt is sent out.

In order to enable tracking for the motion trajectory of the jaw bone to be more accurate, in addition, the jaw bone of the user should be kept at an appropriate distance from the camera, therefore, before the motion trajectory of the identification point is acquired, whether there is any abnormality in the location information of the identification point is confirmed first, for example, a prescanning process is set, first, prescanning is clicked on an interactive interface, then the camera is controlled to acquire three-dimensional coordinate information of the identification point, then three-dimensional coordinates of the identification point acquired by prescanning are compared with three-dimensional coordinates corresponding to position information preset of the identification point to determine whether they are consistent, or a difference value between the acquired three-dimensional coordinates of the identification point and the three-dimensional coordinates corresponding to the position information preset is smaller than an allowable error threshold is judged, etc., the three-dimensional coordinates of the identification point acquired by prescanning may be three-dimensional coordinates of all identification points of the user in a certain tooth-exposed posture, and the preset position information of the identification point may be pre-stored three-dimensional coordinates of the identification points of the lower bone of the user in the same state.

In a case that the comparing result is consistent, the motion trajectory of the identification point may be further acquired, in a case that it is inconsistent, a reminding may be sent to remind the cause of mismatch, for example a posture is incorrect, etc.

In a case that the comparing result is consistent, a first display signal may be sent to a preset display device for displaying green on the display device, then countdown may also be carried out, after countdown, the motion trajectory of the identification point is acquired at once, in a case that the comparing result is inconsistent, a second display signal may be sent to the preset display device for displaying red on the display device.

Based on the above method, the reliability of the motion track trajectory result is promoted, and meanwhile, a distance between the user and the camera is kept within a suitable range.

In an implementation mode of the embodiments of the present disclosure, a motion trajectory of a preset identification point is acquired includes the following operations.

The identification point is scanned to obtain three-dimensional coordinates of the identification point, a motion trajectory of the identification point is determined based on the three-dimensional coordinates of the identification point, and a second jaw bone three-dimensional model based on the three-dimensional coordinates of the identification point and the face of the user is generated.

In some embodiments, when the motion trajectory of the identification point is acquired, first, three-dimensional coordinates of the identification point need to be confirmed, for example, a certain three-dimensional coordinate system is established, then coordinate values of the identification point under the three-dimensional coordinate system are determined, and then the motion trajectory of the identification point is determined according to coordinate values of the identification point under the three-dimensional coordinate system.

Through the above method, the motion trajectory of the identification point and the second jaw bone three-dimensional model may be preliminarily determined, and data is provided for determining the motion trajectory of the jaw bone.

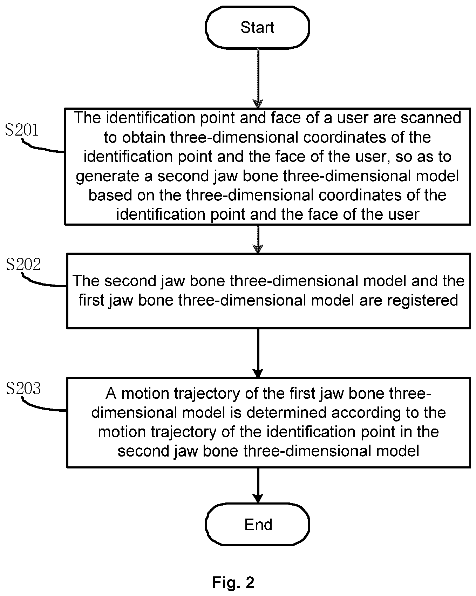

As shown in Fig. 2, Fig. 2 is a detailed flow diagram of S103 in Fig. 1, and in an implementation mode of the embodiments of the present disclosure, S103 may include S201-S203.

In S201, the identification point and face of the user are scanned to obtain three-dimensional coordinates of the identification point and the face of the user, so as to generate a second jaw bone three-dimensional model based on the three-dimensional coordinates of the identification point and the face of the user.

In S202, the second jaw bone three-dimensional model and the first jaw bone three-dimensional model are registered.

In S203, a motion trajectory of the first jaw bone three-dimensional model is determined according to the motion trajectory of the identification point in the second jaw bone three-dimensional model.

With further reference to the example corresponding to Fig. 1, the first jaw bone three-dimensional model is an accurate tooth three-dimensional model determined by the intraoral scanner, but the first jaw bone three-dimensional model is not suitable for directly serving as a jaw bone model for tracking the motion trajectory of the jaw bone, the second jaw bone three-dimensional model is a facial three-dimensional model determined through the preset identification point and the face of the user, which is convenient for tracking the motion trajectory of the jaw bone, therefore, the second jaw bone three-dimensional model may be generated based on the three-dimensional coordinates of the identification point and the face of the user, for example, the identification point and the face of the user are scanned through the facial scanner when the user is in a certain tooth-exposed posture, the three-dimensional coordinates of the identification point and the face of the user are determined, and then the second jaw bone three-dimensional model corresponding to the three-dimensional coordinates of identification point and the face of the user is determined according to the three-dimensional coordinates of the identification point and the face of the user. The first jaw bone three-dimensional model and the second jaw bone three-dimensional model are registered, the model registering method may be any method well known to those skilled in the art, for example, the first jaw bone three-dimensional model and the second jaw bone three-dimensional model may be registered to the same three-dimensional coordinate system, then the motion trajectory of the first jaw bone three-dimensional model is determined according to the motion trajectory of the identification point in the second jaw bone three-dimensional model, points corresponding to the identification point in the second jaw bone three-dimensional model exist in the second jaw bone three-dimensional model, after the motion trajectory of the identification point is determined, the motion trajectory of the point corresponding to the same in the same jaw bone three-dimensional model is determined, since the second jaw bone three-dimensional model and the first jaw bone three-dimensional model have been registered, the motion trajectory of the first jaw bone three-dimensional model may be determined, and thus the motion trajectory of the jaw bone is tracked. In the embodiments of the present disclosure, the sequence of steps in the embodiments of the present disclosure is set to better explain the embodiments of the present disclosure, in an actual implementation process, those skilled in the art may adjust the sequence of some steps without changing the technical solution of the embodiments of the present disclosure for certain considerations, for example, in the actual implementation process, the first jaw bone three-dimensional model and the second jaw bone three-dimensional model do not need to be registered for a plurality of times, therefore, the first jaw bone three-dimensional model and the second jaw bone three-dimensional model may be registered first, then the motion trajectory of the identification point is determined, etc., namely, in an actual process, it may be as follows. 1. A first jaw bone three-dimensional model of a user is acquired. 2. A second jaw bone three-dimensional model of the user is acquired. 3. The first jaw bone three-dimensional model and the second jaw bone three-dimensional model are registered. 4. A motion trajectory of the first jaw bone three-dimensional model is determined according to the motion trajectory of the identification point. A specific procedure may refer to corresponding descriptions in the above embodiment, and no elaboration will be made here for simplicity of description.

The method realizes accurate tracking on the motion trajectory of the jaw bone, for example, on the motion trajectory of movements including left movement, right movement, front stretching, opening and closing, and the like of the lower jaw.

In an implementation mode of the embodiments of the present disclosure, after the operation that the identification point is scanned to obtain three-dimensional coordinates of the identification point, and the second jaw bone three-dimensional model based on the three-dimensional coordinates of the identification point is generated, the method for tracking the motion trajectory of the jaw bone may further include the following operation.

A display signal is sent to a preset display device so that the three-dimensional coordinates of the identification point are displayed by the display device.

After the second jaw bone three-dimensional model is generated, the three-dimensional coordinates of the identification point may also be displayed by a display device, so that a user or others may determine that whether abnormality exists in the generated second jaw bone three-dimensional model.

In addition, displaying the three-dimensional coordinates of the identification point by the display device is not limited to after the second jaw bone three-dimensional model based on the three-dimensional coordinates of the identification point is generated, it may also be that the three-dimensional coordinates of the identification point are displayed by the display device before the second jaw bone three-dimensional model based on the three-dimensional coordinates of the identification point is generated.

In an implementation mode of the embodiments of the present disclosure, after S103, the method may further include the following operation.

A display signal is sent to a preset display device so that the motion trajectory of jaw bone of the user is displayed by the display device.

In order to show the motion trajectory of the jaw bone to the user or others, the above first jaw bone three-dimensional model may also be displayed to the user or others by the display device, so that the motion trajectory of the jaw bone may be more clearly displayed to the user or others.

In an implementation mode of the embodiments of the present disclosure, after S103, the method may further include the following operation.

A display signal is sent to a preset display device so that two-dimensional coordinates of the identification point are displayed by the display device.

Two-dimensional coordinates refer to plane coordinates, by displaying two-dimensional coordinates, a user or others may check whether there is an abnormality in collection of three-dimensional coordinates of the identification point, so as to timely discover and deal with the abnormality in the collection of the three-dimensional coordinates of the identification point.

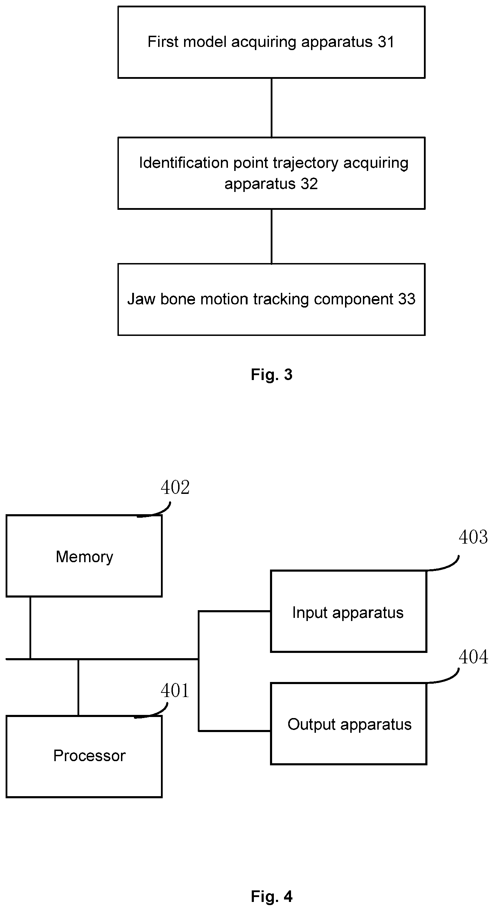

As shown in Fig. 3, Fig. 3 is a schematic structure diagram of an apparatus for tracking a motion trajectory of jaw bone provided by the embodiments of the present disclosure. The apparatus includes a first model acquiring apparatus 31, an identification point trajectory acquiring apparatus 32, and a jaw bone motion tracking component 33.

The first model acquiring apparatus 31 is configured to acquire a first jaw bone three-dimensional model of a user.

The identification point trajectory acquiring apparatus 32 is configured to acquire a motion trajectory of a preset identification point, and the identification point is disposed at jaw bone of the user.

The jaw bone motion tracking component 33 is configured to determine a motion trajectory of the first jaw bone three-dimensional model according to the motion trajectory of the identification point, for tracking the motion trajectory of jaw bone of the user.

The embodiments of the present disclosure further provide an electronic device, which includes a processor and a memory. The processor is configured to perform steps of any one of the methods provided by the above implementation mode by calling a program or instruction stored in the memory, for realizing corresponding beneficial effects.

Fig. 4 is a schematic diagram of a hardware structure of an electronic device provided by the embodiments of the present disclosure. As shown in Fig. 4, the electronic device includes one or more processors 401 and a memory 402.

The processor 401 may be a Central Processing Unit (CPU) or a processing unit in other forms with data processing capabilities and/or instruction execution capabilities, which may control other components in the electronic device to perform the desired function.

The memory 402 may include one or more computer program products, and computer program products may include computer readable storage media of various forms, such as a volatile memory and/or a non-volatile memory. The volatile memory, for example, may include a Random Access Memory (RAM) and/or a cache. The non-volatile memory, for example, may include a Read-Only Memory (ROM), a hard disk, a flash memory and the like. One or more computer program instructions may be stored on the computer readable storage medium, and the processor 401 may run the program instruction to implement the method of embodiments of the present disclosure above, and/or other desired functions. Various contents such as input signal, signal component and noise component may also be stored in the computer readable storage medium.

In an example, the electronic device may further include: an input apparatus 403 and an output apparatus 404, which are interconnected by a bus system and/or connection mechanism (not shown) of other forms.

In addition, the input apparatus 403 may further include a keyboard, a mouse and the like.

The output apparatus 404 may output a various information to the outside, including the determined distance information, direction information, and the like. The output apparatus 404 may include, for example, a display, a loudspeaker, a printer, a communication network and remote output devices to which they are connected.

Of course, for simplicity, Fig. 4 only illustrates some of components relevant to the present disclosure in the electronic device, and components such as a bus and an input/output interface are omitted. In addition, according to a specific application situation, the electronic device may further include any other appropriate component.

The embodiments of the present disclosure further provide a computer-readable storage medium storing a program or instruction, which enables a computer to execute steps of any one of the methods provided by the implementation mode.

In some embodiments, the computer-executable instruction, when executed by a computer processor, may also be configured to execute the technical solution of the method for tracking a motion trajectory of jaw bone provided by the embodiments of the present disclosure, for realizing corresponding beneficial effects.

It is to be noted that in the specification, relational terms such as "first" and "second" are used merely to distinguish one entity or operation from another entity or operation without necessarily requiring or implying any actual such relationship or order between such entities or operations. Terms "include" and "contain" or any other variant thereof is intended to cover nonexclusive inclusions herein, so that a process, method, object or device including a series of elements not only includes those elements but also includes other elements which are not clearly listed or further includes elements intrinsic to the process, the method, the object or the device. An element defined by the statement "comprises a..." does not, without more constraints, preclude the existence of additional identical elements in the process, method, article, or devices that comprises the element.

The above are only specific implementation modes of the present disclosure to enable those skilled in the art to understand or implement the present disclosure. Various modifications to these embodiments will be readily apparent to those skilled in the art, and the generic principles defined herein may be implemented in other embodiments without departing from the spirit or scope of the present disclosure. Thus, the present disclosure is not to be limited to these embodiments shown herein but is to be accorded the widest scope consistent with the principles and novel features disclosed herein.

### Industrial Applicability

The jaw bone motion tracking method provided by the present disclosure realizes tracking for jaw bone motion of the user under the premise that the identification point is disposed at jaw bone of the user, so that use difficulty is reduced, the device is easy to mount, meanwhile, the jaw bone motion is tracked only through the identification point, so interference caused on jaw bone motion by a corresponding tracking apparatus by using a traditional jaw bone motion tracking method is avoided, influence on the motion trajectory of the jaw bone is reduced, and the industrial applicability is very strong.

## Claims

1. A method for tracking a motion trajectory of jaw bone, comprising:
acquiring a first jaw bone three-dimensional model of a user;
acquiring a motion trajectory of a preset identification point, wherein the identification point is disposed at jaw bone of the user;
determining a motion trajectory of the first jaw bone three-dimensional model according to the motion trajectory of the identification point, for tracking motion trajectory of jaw bone of the user.

2. The method for tracking the motion trajectory of jaw bone as claimed in claim 1, before acquiring the motion trajectory of the preset identification point, further comprising:
acquiring position information of the identification point, and judging whether the position information of the identification point is matched with preset position information of the identification point;
in a case that the position information of the identification point is matched with preset position information of the identification point, executing the step of acquiring a motion trajectory of a preset identification point;
in a case that the position information of the identification point is not matched with preset position information of the identification point, sending out a prompt.

3. The method for tracking the motion trajectory of jaw bone as claimed in claim 2, wherein acquiring the motion trajectory of the preset identification point comprises:
scanning the identification point to obtain three-dimensional coordinates of the identification point, and determining the motion trajectory of the identification point based on the three-dimensional coordinates of the identification point.

4. The method for tracking the motion trajectory of jaw bone as claimed in claim 3, wherein determining the motion trajectory of the first jaw bone three-dimensional model according to the motion trajectory of the identification point comprises:
scanning the identification point and face of the user to obtain three-dimensional coordinates of the identification point and the face of the user, so as to generate a second jaw bone three-dimensional model based on the three-dimensional coordinates of the identification point and the face of the user;
registering the second jaw bone three-dimensional model and the first jaw bone three-dimensional model;
determining a motion trajectory of the first jaw bone three-dimensional model according to the motion trajectory of the identification point in the second jaw bone three-dimensional model.

5. The method for tracking the motion trajectory of jaw bone as claimed in claim 3, after scanning the identification point to obtain three-dimensional coordinates of the identification point, and determining the motion trajectory of the identification point based on the three-dimensional coordinates of the identification point, further comprising:
sending a display signal to a preset display device so that the three-dimensional coordinates of the identification point are displayed by the display device.

6. The method for tracking the motion trajectory of jaw bone as claimed in claim 1, after determining the motion trajectory of the first jaw bone three-dimensional model according to the motion trajectory of the identification point, for tracking motion trajectory of jaw bone of the user, further comprising:
sending a display signal to a preset display device so that the motion trajectory of jaw bone of the user is displayed by the display device.

7. The method for tracking the motion trajectory of jaw bone as claimed in claim 1, after determining the motion trajectory of the first jaw bone three-dimensional model according to the motion trajectory of the identification point, for tracking motion trajectory of jaw bone of the user, further comprising:
sending a display signal to a preset display device so that the three-dimensional coordinates of the identification point are displayed by the display device.

8. An apparatus for tracking a motion trajectory of jaw bone, comprising:
a first model acquiring apparatus, which is configured to acquire a first jaw bone three-dimensional model of a user;
an identification point trajectory acquiring apparatus, which is configured to acquire a motion trajectory of a preset identification point, wherein the identification point is disposed at jaw bone of the user;
a jaw bone motion tracking component, which is configured to determine a motion trajectory of the first jaw bone three-dimensional model according to the motion trajectory of the identification point, for tracking the motion trajectory of jaw bone of the user.

9. An electronic device, comprising a processor and a memory;
the processor is configured to execute steps of the method as claimed in any of claims 1-7 by calling a program or instruction stored in the memory.

10. A computer-readable storage medium storing a program or instruction, which enables a computer to execute steps of the method as claimed in any of claims 1-7.
